# EUROPÄISCHE PATENTANMELDUNG

(11) **EP 4 585 170 A1**
(43) Veröffentlichungstag der Anmeldung: **16.07.2025**
(21) Anmeldenummer: 24183120.5
(22) Anmeldetag: 19.06.2024
(51) Int. Cl.: A61B 17/22, A61B 17/225, A61B 17/00

(54) **DRUCKWELLENGERÄT MIT VERBESSERTEM FÜHRUNGSROHR**

(30) Priorität: 12.01.2024 EP 24151649
(71) Anmelder: STORZ MEDICAL AG, 8274 Tägerwilen (CH)
(72) Erfinder: Storz, Rafael, Kreuzlingen (CH); Belau, Markus, Konstanz (DE); Kühl, Arvid, Tägerwilen (CH); Gremlich, Felix, Bottighofen (CH); König, Janis, Kreuzlingen (CH)
(74) Vertreter: Szynka Smorodin Patentanwälte Partnerschaft mbB

(57) **Zusammenfassung**

Die Erfindung betrifft eine Vorrichtung zur Behandlung eines menschlichen oder tierischen Körpers mit mechanischen Druckwellen, welche Vorrichtung aufweist: ein formstabiles Führungsrohr, ein in dem Führungsrohr bewegliches Projektil, eine Einrichtung zum Beschleunigen des Projektils in dem Führungsrohr zu der Bewegung und einen Applikator an einem Ende des Führungsrohres zum Aufprall des beschleunigten Projektils darauf zur Erzeugung der Druckwellen und zum Einkoppeln der Druckwellen in den Körper, dadurch gekennzeichnet, dass das Führungsrohr mindestens innenseitig aus einem Material mit einer Härte von höchstens 150HV0,1 nach der Vickers-Skala besteht.

## Beschreibung

Die Erfindung betrifft ein Gerät zum Behandeln des menschlichen oder tierischen Körpers mittels mechanischer Druckwellen.

Solche Vorrichtungen sind an sich bereits bekannt, insbesondere aus dem Bereich der Lithotripsie. Dort werden mit fokussierten mechanischen Druckwellen Körperkonkremente, insbesondere Steine im Körpergewebe, zertrümmert. Ferner sind Geräte entwickelt worden, die mechanische Druckwellen durch das Aufeinanderprallen eines beschleunigten Projektils und eines quasi als Amboss fungierenden Applikators erzeugen und über den Applikator in zu behandelndes Körpergewebe einkoppeln. Vorrangig sind solche Geräte zur Einkopplung nicht fokussierter Druckwellen ausgelegt und werden bspw. zur Behandlung von Muskelerkrankungen und von Erkrankungen im Übergangsbereich zwischen Muskeln und Knochen und vielen anderen Indikationen eingesetzt.

Die gerade beschriebene Vorrichtungsgattung kann bezeichnet werden als "ballistische Druckwellengeräte" und erzeugt typischerweise etwas weniger energiereiche Druckwellen mit nicht ganz so steiler anfänglicher Druckanstiegsflanke wie eigentliche Lithotripsiegeräte. Im Übrigen sind bei der Einkopplung der Druckwellen zwei Mechanismen zu unterscheiden. Einerseits verlagert sich der typischerweise elastisch in dem Druckwellengerät aufgehängte Applikator durch die Kollision "makroskopisch", also im Sinn einer Schwerpunktbewegung. Daher erzeugt ein unmittelbar oder mittelbar auf dem zu behandelnden Körper aufliegender Applikatorteil eine entsprechende Druckwelle. Andererseits entsteht in dem Applikator infolge der Kollision eine deutlich höherfrequente Schallwelle (typischerweise 75-200 kHz), die sich durch den Applikator fortpflanzt und von diesem in das Körpergewebe eingekoppelt wird.

Beide Druckwellen können therapeutische Effekte haben. In der Regel steht in der Literatur die hochfrequente Welle, also der zweite beschriebene Mechanismus, im Vordergrund.

Zum Stand der Technik wird stellvertretend verwiesen auf das Patent EP 2 213 273 B1 der Anmelderin, das ein typisches Gerät zeigt und sich mit Einzelheiten einer pneumatischen Einrichtung zur Beschleunigung des Projektils (dort als Schlagteil bezeichnet) befasst.

Auf dieser Grundlage liegt der Erfindung die Aufgabe zugrunde, ein solches Druckwellengerät technisch weiterzuentwickeln.

Das für die Führung des Projektils vorgesehene Führungsrohr einschlägiger Vorrichtungen aus dem Stand der Technik besteht aus korrosionsfreiem Stahl (VA-Stahl). Das Gleiche gilt für das Projektil, wobei in der Praxis zur Verbesserung der Lebensdauer des Projektils eine Hartbeschichtung aus einem Karbid eingesetzt wurde. Außerdem wurde das Führungsrohr bei einem Innendurchmesser von typischerweise 6 mm innenwandig präzisionsbearbeitet, etwa mit einer Toleranz von 0,01 mm.

Erfindungsgemäß wird nun ein deutlich weicheres Führungsrohrmaterial benutzt, jedenfalls auf der Innenseite des Führungsrohres, mit der das Projektil in Kontakt kommt. Konkret soll das hier verwendete Material eine Härte nach der Vickers-Skala von höchstens 150HV0,1 haben, vorzugsweise höchstens 120HV0,1 oder höchstens 100HV0,1. Weitere bevorzugte Obergrenzen sind 80HV0,1, 60HV0,1 und sowie 40HV0,1. Damit können verschiedene Vorteile erzielt werden.

Maßgeblich ist hier die aktuelle EN ISO 6507-1:2023.

Zum einen kann vorzugsweise auf die Hartbeschichtung des Projektils verzichtet werden. Das verringert den technischen Aufwand und die Kosten. Es bringt aber auch Vorteile für die Betriebssicherheit und Dauerhaftigkeit, weil sich bei beschichtetem Projektil nach längeren Laufzeiten Abplatzungen der Beschichtung ergaben und abgeplatzte Stücke den Verschleiß erhöhen oder sogar zu Blockaden führen konnten. Bei erfindungsgemäß weicherem Material der Innenwand des Führungsrohres kommt auch ein unbeschichtetes Projektil in Betracht, insbesondere aus Metall, vorzugsweise aus VA-Stahl.

Zusätzlich oder alternativ können einfache und kostengünstige Führungsrohre aus z. B. Polymerwerkstoff benutzt werden, insbesondere aus thermoplastischem Polymerwerkstoff. Bevorzugte Möglichkeiten sind PTFE (Polytetrafluorethylen), PET (Polyethylenterephthalat), POM (Polyoxymethylen) und besonders Polyamide, insbesondere PA6. In Betracht kommen auch faserverstärkte Kunststoffe, insbesondere glasfaserverstärkte Kunststoffe.

Im Prinzip reicht es, wenn die Innenwände des Führungsrohres aus solchem oder anderem weichen Werkstoff bestehen. Die Führungsrohre können auch aus Vollmaterial bestehen; es ist also keine Umhüllung z. B. aus Stabilitätsgründen notwendig (aber natürlich möglich). Damit kann z. B. extrudiertes Rohrmaterial eingesetzt werden, das ganz erheblich viel günstiger ist als präzisionsbearbeitetes VA-Rohr. Es können aber auch geeignete Rohre mit einer Bohrung und optional nachträglichem Durchstoßen mit scharfem Stempel auf ein geeignetes Innenmaß gebracht und erfindungsgemäß verwendet werden.

Die Materialänderung bedingt nicht zwingend Unterschiede in den geometrischen Maßen. So sind für die dem Ausführungsbeispiel zugrunde liegenden Versuche Polyamidrohre mit Maßen eingesetzt worden, die dem zuvor eingesetzten VA-Rohr entsprachen. Typische Wandstärken liegen dabei dementsprechend im Bereich von 0,5 mm bis 2,5 mm wobei als Untergrenze 0,6 mm, 0,7 mm und 0,8 mm sowie als Obergrenze 2,0 mm, 1,7 mm und 1,4 mm zunehmend bevorzugt sind.

Jedenfalls hat sich herausgestellt, dass durch das vergleichsweise sehr weiche Führungsrohr sehr gute Laufeigenschaften und eine überraschend sehr gute

Dauerhaftigkeit erreicht werden konnte. Es kommt offenbar nicht vorrangig darauf an, für die Führungsrohrinnenwand hartes Material zu verwenden.

Konkret sind die bislang gebräuchlichen VA-Führungsrohre genauso Verschleißartikel wie Projektile und Applikatoren. Überraschenderweise konnte die Lebensdauer der Führungsrohre in der beschriebenen Weise weit über die typische Einsatzdauer eines konventionellen VA-Führungsrohres hinaus erhöht werden.

Vorzugsweise ist das Führungsrohr für sich formstabil. Das bedeutet, dass es sich unter seinem Eigengewicht nicht in einer praktisch relevanten Weise verformt, bspw. bei einer typischen Länge von 10 cm und einseitiger Einspannung um nicht mehr als 0,5 mm, vorzugsweise nicht mehr als 0,3 mm, 0,2 mm oder sogar nicht mehr als 0,1 mm verformt, also um höchstens 0,5 %, 0,3 %, 0,2 % bzw. 0,1 % der Länge. Das gilt entsprechend für andere Längen. In einfacheren Worten: Es handelt sich bei dem Führungsrohr um ein Rohrstück und nicht um ein Schlauchstück. Diese Anforderungen lassen sich z. B. mit den erwähnten Polymermaterialien problemlos realisieren.

Die Formstabilität vermeidet die Notwendigkeit, durch eine besondere justierende Halterung für die gewünschte Geradheit des Führungsrohres zu sorgen. Bei nicht formstabilen Schlauchstücken bestünde die Gefahr, dass eine ungewollte Verformung zu Abweichungen von der eigentlich für das Innenvolumen gewünschten Bahn führt, was für die Projektilbewegung grundsätzlich nachteilig wäre und höheren Projektilgeschwindigkeiten und- frequenzen im Wege stünde.

Ferner ist bevorzugt, dass das Projektilmaterial deutlich härter als das Material der Innenwand des Führungsrohres ist. Dementsprechend ist die Härte des Projektils zumindest an seiner mit dem Führungsrohr in Kontakt kommenden Außenmantelfläche vorzugsweise größer als 170HV0,1, wobei folgende Untergrenzen zunehmend bevorzugt sind: 180HV0,1, 190HV0,1 und schließlich 200HV0,1. In Betracht kommen neben Metallen, insbesondere Stahl, auch Keramiken und Verbundmaterialien. Dabei sind nicht zwingend besonders harte Metalle notwendig, sondern können sich durch das erfindungsgemäße Führungsrohr auch Anwendungsmöglichkeiten für etwas weichere Metalle ergeben. Die Versuche der Erfinder haben nämlich einen erheblich geringeren Verschleiß auch der Projektile gezeigt, nämlich einen viel geringeren Abrieb der mit der Führungsrohrinnenwand in Kontakt kommenden Außenwandbereiche des Projektils. Insbesondere ist in diesem Zusammenhang schon aus ökonomischen Gründen von Vorteil, auf im Stand der Technik übliche Hartbeschichtungen von Projektilen zu verzichten.

Ferner sind die erfindungsgemäß geeigneten Materialien für das Führungsrohr typischerweise deutlich leichter als Metalle, insbesondere VA-Stahl. In diesem Sinn sind für das Führungsrohr (mindestens innenseitig, vorzugsweise überhaupt) Dichten von höchstens 3 kg/l bevorzugt, wobei die folgenden Obergrenzen zunehmend bevorzugt sind: 2,5 kg/l, 2,0 kg/l und schließlich 1,5 kg/l. Es ist generell von Vorteil, Gewicht einzusparen, was bei den im vorliegenden Fall gebräuchlichen und auch bevorzugten Ausführungen mit einem von einem Basisgerät getrennten Handstück in besonderem Maße gilt.

Vorzugsweise ist das erfindungsgemäße Druckwellengerät dazu ausgelegt, mit dem Applikator von außen auf eine Hautpartie eines Patienten aufgesetzt zu werden. In anderen Worten handelt es sich um ein extrakorporal einzusetzendes Gerät im Unterschied zu aus dem Stand der Technik zu Lithotriptern bekannten Geräten, die über lange dünne Applikatoren, die im Regelfall durch Katheter o. ä. eingeführt werden, Körperkonkremente zertrümmern. Bei der Anwendung von außen wird eine Einkopplung der therapeutisch gewünschten Druckwellen in den Körper bewirkt, wobei z. B. zur Impedanzanpassung ein Kontaktmedium (Ultraschallgel etwa) benutzt werden kann.

Ferner kann der Applikator aus Impedanzgründen mehrteilig sein, also um die für die Druckwellen relevante Impedanz des Applikators im Bereich der Kollision mit dem Projektil besser anzupassen an die Impedanz des Kontaktmediums oder des Körpergewebes.

Eine typische Geometrie einer für das Aufsetzen günstigen Außenfläche des Applikators zeigt das Ausführungsbeispiel. Es geht also um flächige und zur Vermeidung von Verletzungen in diesem Sinn nicht zu kleine Kontaktflächen von typischerweise mindestens 1 cm² oder mehr.

Die Führungsrohrlänge kann günstiger Weise begrenzt werden auf einen Bereich von z. B. ca. 5-20 cm. Eine solche Länge reicht für eine effiziente Beschleunigung aus und erlaubt andererseits eine kompakte Baugröße. Das gilt insbesondere bei der hier bevorzugten vollständigen Unterbringung des Führungsrohres in einem Gerätegehäuse. Ein solches Gerätegehäuse kann ein Handstück sein, das von einer Basisstation mit pneumatischem Druck versorgt wird, wobei die Basisstation weniger mobil ist (bspw. auf einem Wagen steht) und das Handstück von der behandelnden Person mit der Hand geführt wird.

In diesem Zusammenhang ist bevorzugt, dass das Handstück auch ein pneumatisches Schaltventil enthält, also die für die Beschleunigung zuständigen pneumatischen Pulse durch eine Ventilaktion in dem Gehäuse oder an dessen Rand, jedoch nicht im Basisgerät oder am anderen Ende der Versorgungsleitung, erzeugt werden.

Der bereits erwähnte Applikator ist vorzugsweise in mindestens einem Elastomerring elastisch gehalten und damit von dem Gehäuse oder einer anderen Aufhängung entkoppelt. Das ist sinnvoll, um die Druckwellen in nicht zu großem Maß in das Gehäuse einzukoppeln. Andererseits erlaubt der Elastomerring nur eine begrenzte Bewegung (im Sinne von Schwerpunktbewegung, also Gesamtbewegung) des Applikators. Im Ausführungsbeispiel ist das deutlich zu sehen mit einer Mehrzahl Elastomerringe.

Typische erreichbare Projektilgeschwindigkeiten können im Bereich zwischen 5 m/s und 60 m/s liegen. Dabei sind vorzugsweise Wiederholfrequenzen erreichbar von mindestens 10 Hz, vorzugsweise mindestens 20 Hz oder sogar mindestens 25 Hz.

Die Rückbewegung des Projektils vor der neuen Beschleunigung kann z. B. durch die Kollision mit dem Applikator und/oder auch durch eine im Stand der Technik bereits bekannte Gegendruckkammer (wie beim Ausführungsbeispiel) oder auch in anderer Weise erreicht werden.

Ein weiterer Vorteil der Erfindung kann sich bei der Verwendung von Sensoren zur Positions- und/oder Geschwindigkeitserfassung des Projektils ergeben. Hier sind insbesondere Hall-Sensoren gut geeignet und werden durch ein nicht metallisches Führungsrohr-Material deutlich weniger behindert, insbesondere durch einen Polymerwerkstoff.

Außerdem kommen optische Erfassungsvorrichtungen in Betracht und der Einsatz von Polymerwerkstoffen erlaubt grundsätzlich auch transparente Materialien, z. B. transparentes Polyamid.

Wie schon in dem zitierten Stand der Technik ist im vorliegenden Zusammenhang eine pneumatische Einrichtung zur Beschleunigung des Projektils bevorzugt. Eine solche Beschleunigungseinrichtung beaufschlagt typischerweise ein zu dem Applikator entgegengesetztes Ende des Führungsrohres mit Druck zur Beschleunigung. Sie ist aber nicht notwendig; das Projektil könnte auch z. B. elektromagnetisch beschleunigt werden, insbesondere durch eine um das Führungsrohr herum gewickelte Spule in Verbindung mit einem ferromagnetischen Projektil. Möglich sind auch wandernde Magnetfelder in Verbindung mit ferromagnetischen Projektilen.

Die Erfindung betrifft auch die Verwendung eines Rohres als Führungsrohr für eine einschlägige Vorrichtung sowie die Verwendung eines Projektils hierfür, insbesondere eines unbeschichteten Metallprojektils und vorzugsweise unbeschichteten VA-Projektils.

Im Folgenden wird die Erfindung anhand eines Ausführungsbeispiels näher erläutert, das sich auf alle Anspruchskategorien bezieht und dessen einzelne Merkmale auch in anderen Kombinationen wesentlich sein können.

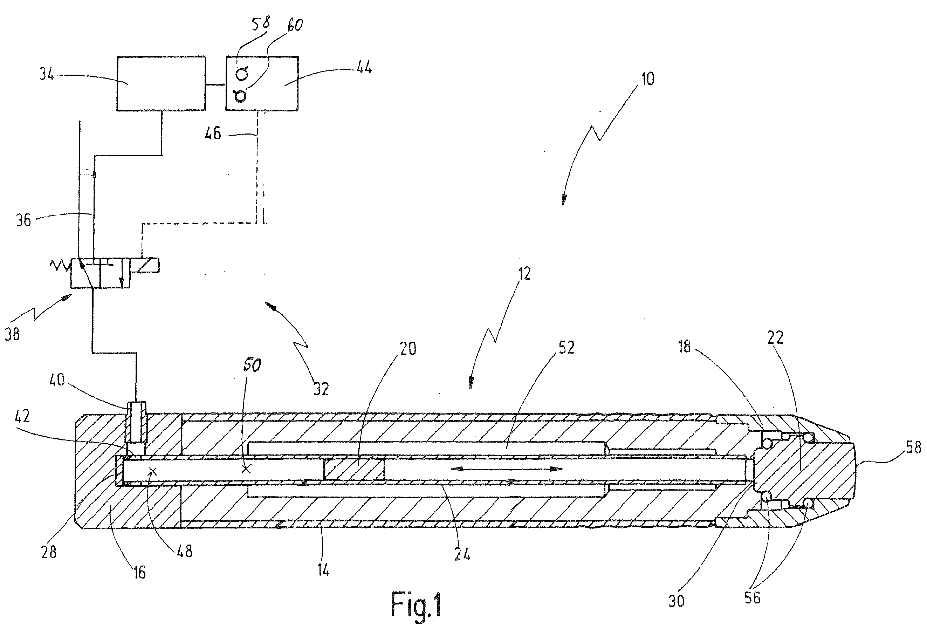

- Figur 1: zeigt ein erfindungsgemäßes Gerät im Längsschnitt mit schematisch dargestelltem pneumatischen Antrieb.

**Figur 1** zeigt ein insgesamt mit 10 bezeichnetes medizinisches Gerät zur Behandlung des menschlichen Körpers mit mechanischen Druckwellen, und zwar in diesem Fall zur Weichgewebebehandlung im Rahmen einer Schmerztherapie. Das Gerät besteht aus einem Handstück 12 und einer weiter unten näher erläuterten pneumatischen Druckgasversorgungseinrichtung 32. Ein behandelnder Arzt beispielsweise kann das Handstück 12 greifen und auf eine geeignete Hautpartie mit dem in Figur 1 rechten Ende aufsetzen, wobei das Handstück 12 ungefähr senkrecht auf der Haut steht.

Ein Gehäuse 14 ist mit einer proximalen Endkappe 16 und distalen Endkappe 18 versehen, die jeweils abnehmbar ausgeführt sind. In dem Gehäuse ist ein Führungsrohr 24 gehalten und dabei axial und konzentrisch angeordnet. In dem Führungsrohr ist ein Projektil 20 geführt, dessen Bewegungsstrecke entlang des Innenraums des Führungsrohrs 24 auf der rechten Seite durch einen Applikator 22 begrenzt ist, und zwar durch dessen proximale Seite 30. Diese bildet einen distalen Anschlag des Projektils 20, wobei der proximale Anschlag des Projektils 20 mit 28 bezeichnet ist und einen einfachen Abschluss des Führungsrohres 24 bildet. Dieser Abschluss ist magnetisch, sodass das Projektil 20 mit einer gewissen Haltekraft dort fixiert sein kann. Typischerweise beträgt die Länge des Führungsrohres 24 etwa 5 cm - 20 cm, wobei das hier gezeichnete Ausführungsbeispiel eine Führungsrohrlänge von 16 cm hat.

Der pneumatische Antrieb 32 verkörpert die Druckgasversorgungseinrichtung und weist einen üblichen pneumatischen Kompressor 34 (oder eine Druckgasflasche) auf, wobei der Kompressor 34 einen typischen Arbeitsbereich bis etwa 10 bar abdeckt. Über eine Druckleitung 36 und ein Schaltventil 38 wird ein Druckgasanschluss 40 des Handstücks 12 versorgt, der mit dem Führungsrohr 24 über eine Öffnung 42 darin kommuniziert. Bei dem Schaltventil 38 kann es sich um ein Magnetventil handeln. Eine Steuerung 44 ist mit diesem über eine Steuerleitung 46 verbunden, die gestrichelt dargestellt ist. Die Steuerung 44 kann mit dem Kompressor 34 als Baueinheit ausgeführt sein und damit ein Basisgerät zur Versorgung des Handstücks 12 bilden, wobei an letzterem günstigerweise das Schaltventil 38 angebracht ist. Das hat den Vorteil, dass das von dem Druckpuls zu befüllende Volumen klein wird. Dadurch werden stärkere und schnellere Impulse realisierbar. Dementsprechend sind die Steuerung 44 und der Kompressor 34 in Figur 1 durch eine Leitung verbunden. Basisgerät und Handstück 12 sind dann über eine die Pneumatikleitung 36 und die Steuerleitung 46 zusammenfassende Versorgungsleitung verbunden.

Ausgehend von einem Ruhezustand des Geräts 10, also beim Betriebsstart, wird das geschlossene Schaltventil 38 durch die Steuerung 44 geöffnet. Der in Figur 1 dargestellte Zustand, in dem das Führungsrohr 24 mit der Außenatmosphäre verbunden ist, wird damit zu einem Zustand geändert, der mit dem rechten Kästchen des Ventilsymbols dargestellt ist, wobei der Versorgungsdruck über den Anschluss 40 an das Führungsrohr 24 angelegt wird. Dabei befindet sich das Projektil 20 zunächst in seiner Ausgangsstellung, die in Figur 1 mit 48 angedeutet ist. Der aufgebaute Druck beschleunigt das Projektil 20 in Richtung des Prallkörpers, wird aber noch vor dem Aufprall durch Wiederzurückschalten des Schaltventils 38 und damit Belüften des "hinter" dem Projektil 20 befindlichen Volumens in dem Führungsrohr 24 abgebaut. Das Projektil 20 trifft ungebremst auf den Prallkörper 22, dessen distale (leicht konvexe) Abschlussfläche 58 auf der Haut des Patienten aufliegt und eine mechanische Druckwelle in den Körper überträgt. Dabei führt der Applikator 22 infolge seiner elastischen Aufhängung in den beiden Elastomer-O-Ringen 56 eine axiale Bewegung aus.

Unmittelbar nach dem Aufprall bewegt sich das Projektil 20 zurück. Dabei hilft eine Gegendruckkammer 52, die mit dem Führungsrohr 24, und zwar seinem distalen Ende kurz vor der proximalen Seite 30 des Prallkörpers 22, in hier nicht näher dargestellter Weise verbunden ist. In dieser Gegendruckkammer entsteht durch die Verdrängung infolge der Bewegung des Projektils 20 ein das Projektil 20 nach dem Aufprall bis zu dem proximalen Anschlag, also dem magnetischen Abschlussstück 28, zurückführender Gegendruck.

Nach einer bestimmten Zeit wird das Schaltventil 38 wieder umgeschaltet, sodass einer neuer Auslösevorgang beginnt. Diese bestimmte Zeit ergibt zusammen mit der Einschaltzeit des Schaltventils 38 den Kehrwert der eingestellten Frequenz. Typische Aufprallgeschwindigkeiten des Projektils liegen im Bereich von 5 m/s-60 m/s. Im Unterschied zu dem hier in Bezug genommenen Dokument aus dem Stand der Technik besteht das Führungsrohr 24 aus einem extrudierten und/oder durch Bohren und Nachbearbeitung (mit einem scharfen Stempel) auf das richtige Innenmaß gebrachten Polyamid-Rohr. Konkret wurde das Polyamid PA6 verwendet, das nach Messungen eine Härte von etwa 16HV0,1 hat. In diesem Bereich sind solche Messungen mit einer gewissen Streuung behaftet. Konkret handelt es sich um einen Mittelwert bei einer Streuung zwischen 14 und 18HV0,1. Jedenfalls liegt dieser Wert ganz erheblich unter den genannten Obergrenzen.

Die Länge beträgt, wie schon angegeben, 160 mm bei einer Wandstärke von 1 mm, einem Innendurchmesser von 6 mm und demzufolge einem Außendurchmesser von 8 mm. Das verwendete Projektil ist geringfügig kleiner, hat nämlich einen maximalen Außendurchmesser von 5,96 mm.

Das Projektil 20 besteht aus unbeschichtetem VA-Stahl. Damit ergeben sich Vickers-Härten von etwa ab 200HV0,1. Gehärteter VA-Stahl liegt bei über 400HV0,1, wobei die konventionell verwendete Oberflächenbeschichtung diesen Wert erfahrungsgemäß noch erhöht. Wegen ihrer Schichtcharakteristik lässt sie sich an sich schlecht charakterisieren, aber schon an unbeschichteten Stirnflächen steigt durch die Beschichtung die Vickers-Härte auf über 500HV0,1, um ein Beispiel zu nennen. Die Dichte des Führungsrohres 24 liegt im Unterschied zum konventionellen Stahl (mit knapp 8 kg/l) bei nur rund 1,1-1,2 kg/l, typischerweise 1,14 kg/l, also bei gut 1/7. In ersten Versuchen konnten ohne weiteres Lebensdauern des Führungsrohres von weit über 15 Mio. Pulsen erreicht werden.

## Patentansprüche

1. Vorrichtung (10) zur Behandlung eines menschlichen oder tierischen Körpers mit mechanischen Druckwellen, welche Vorrichtung aufweist:
ein formstabiles Führungsrohr (24),
ein in dem Führungsrohr (24) bewegliches Projektil (20),
eine Einrichtung (34-46, 58) zum Beschleunigen des Projektils (20) in dem Führungsrohr (24) zu der Bewegung und
einen Applikator (22) an einem Ende des Führungsrohres (24) zum Aufprall des beschleunigten Projektils (20) darauf zur Erzeugung der Druckwellen und zum Einkoppeln der Druckwellen in den Körper,
**dadurch gekennzeichnet, dass** das Führungsrohr (24) mindestens innenseitig aus einem Material mit einer Härte von höchstens 150HV0,1 nach der Vickers-Skala besteht.

2. Vorrichtung (10) nach Anspruch 1, bei welcher der Applikator (22) zum Aufsetzen auf eine Hautpartie eines Patienten ausgelegt ist.

3. Vorrichtung (10) nach Anspruch 1 oder 2, bei welcher das Führungsrohr (24) eine Länge zwischen 5 cm und 20cm aufweist.

4. Vorrichtung (10) nach einem der vorstehenden Ansprüche mit einem Gehäuse (14) in welchem Gehäuse (14) das gesamte Führungsrohr (24) und das Projektil (20) angeordnet sind.

5. Vorrichtung (10) nach einem der vorstehenden Ansprüche, bei welcher der Applikator (22) mittels zumindest einem Elastomerring elastisch aufgehängt ist.

6. Vorrichtung (10) nach einem der vorstehenden Ansprüche, bei welcher das mindestens innenseitige Material des Führungsrohres (24) ein Polymerwerkstoff ist, insbesondere Polyamid.

7. Vorrichtung (10) nach einem der vorstehenden Ansprüche, bei welcher das mindestens innenseitige Material eine Dichte von höchstens 3 kg/l hat.

8. Vorrichtung (10) nach einem der vorstehenden Ansprüche, bei welcher das Führungsrohr (24) aus dem Material als Vollmaterial besteht.

9. Vorrichtung (10) nach einem der vorstehenden Ansprüche, bei welcher das Projektil (20) aus einem Material mit einer Härte von mindestens 170HV0,1 nach der Vickers-Skala, insbesondere aus Metall, besteht, insbesondere aus VA-Stahl.

10. Vorrichtung (10) nach einem der vorstehenden Ansprüche, bei welcher das Projektil (20) unbeschichtet ist.

11. Vorrichtung (10) nach einem der vorstehenden Ansprüche mit einer Positionserfassungsvorrichtung, insbesondere mit mindestens einem Hall-Sensor zur Positionserfassung des Projektils und/oder mindestens einer optischen Erfassungsvorrichtung zur Erfassung der Position des Projektils (20), wobei das Führungsrohr (24) transparent ist.

12. Vorrichtung (10) nach einem der vorstehenden Ansprüche, bei welcher die Beschleunigungseinrichtung (34-46, 58) eine pneumatische Einrichtung ist, die durch Druckbeaufschlagung des Führungsrohres (24) das Projektil (20) beschleunigen kann.

13. Vorrichtung (10) nach einem der vorstehenden Ansprüche, welche ein Handstück (12) mit dem Führungsrohr (24), dem Projektil (20) und dem Applikator (22) aufweist, welches von einer Bedienperson bei der Behandlung mit der Hand gehalten und geführt werden kann, sowie ein Basisgerät zur Versorgung des Handstücks (12).

14. Verwendung eines Rohres mit einem zumindest innenseitigen Material einer Härte von höchstens 150HV0,1 nach der Vickers-Skala als Führungsrohr (24) für eine Vorrichtung (10) nach einem der vorstehenden Ansprüche.

15. Verwendung eines Projektils (20) für eine Vorrichtung (10) nach einem der Ansprüche 1-13, insbesondere eines Metallprojektils und vorzugsweise eines unbeschichteten VA-Stahl-Projektils.

## Geänderte Patentansprüche

### Geänderte Patentansprüche gemäss Regel 137(2) EPÜ.

1. Vorrichtung (10) zur Behandlung eines menschlichen oder tierischen Körpers mit mechanischen Druckwellen, welche Vorrichtung aufweist:
ein formstabiles Führungsrohr (24),
ein in dem Führungsrohr (24) bewegliches Projektil (20),
eine Einrichtung (34, 36, 38, 40, 42, 44, 46, 58) zum Beschleunigen des Projektils (20) in dem Führungsrohr (24) zu der Bewegung und
einen Applikator (22) an einem Ende des Führungsrohres (24) zum Aufprall des beschleunigten Projektils (20) darauf zur Erzeugung der Druckwellen und zum Einkoppeln der Druckwellen in den Körper,
**dadurch gekennzeichnet, dass** das Führungsrohr (24) mindestens innenseitig aus einem Material mit einer Härte von höchstens 150HV0,1 nach der Vickers-Skala besteht.

2. Vorrichtung (10) nach Anspruch 1, bei welcher der Applikator (22) zum Aufsetzen auf eine Hautpartie eines Patienten ausgelegt ist.

3. Vorrichtung (10) nach Anspruch 1 oder 2, bei welcher das Führungsrohr (24) eine Länge zwischen 5 cm und 20cm aufweist.

4. Vorrichtung (10) nach einem der vorstehenden Ansprüche mit einem Gehäuse (14) in welchem Gehäuse (14) das gesamte Führungsrohr (24) und das Projektil (20) angeordnet sind.

5. Vorrichtung (10) nach einem der vorstehenden Ansprüche, bei welcher der Applikator (22) mittels zumindest einem Elastomerring elastisch aufgehängt ist.

6. Vorrichtung (10) nach einem der vorstehenden Ansprüche, bei welcher das mindestens innenseitige Material des Führungsrohres (24) ein Polymerwerkstoff ist, insbesondere Polyamid.

7. Vorrichtung (10) nach einem der vorstehenden Ansprüche, bei welcher das mindestens innenseitige Material eine Dichte von höchstens 3 kg/l hat.

8. Vorrichtung (10) nach einem der vorstehenden Ansprüche, bei welcher das Führungsrohr (24) aus dem Material als Vollmaterial besteht.

9. Vorrichtung (10) nach einem der vorstehenden Ansprüche, bei welcher das Projektil (20) aus einem Material mit einer Härte von mindestens 170HV0,1 nach der Vickers-Skala, insbesondere aus Metall, besteht, insbesondere aus VA-Stahl.

10. Vorrichtung (10) nach einem der vorstehenden Ansprüche, bei welcher das Projektil (20) unbeschichtet ist.

11. Vorrichtung (10) nach einem der vorstehenden Ansprüche mit einer Positionserfassungsvorrichtung, insbesondere mit mindestens einem Hall-Sensor zur Positionserfassung des Projektils und/oder mindestens einer optischen Erfassungsvorrichtung zur Erfassung der Position des Projektils (20), wobei das Führungsrohr (24) transparent ist.

12. Vorrichtung (10) nach einem der vorstehenden Ansprüche, bei welcher die Beschleunigungseinrichtung (34-46, 58) eine pneumatische Einrichtung ist, die durch Druckbeaufschlagung des Führungsrohres (24) das Projektil (20) beschleunigen kann.

13. Vorrichtung (10) nach einem der vorstehenden Ansprüche, welche ein Handstück (12) mit dem Führungsrohr (24), dem Projektil (20) und dem Applikator (22) aufweist, welches von einer Bedienperson bei der Behandlung mit der Hand gehalten und geführt werden kann, sowie ein Basisgerät zur Versorgung des Handstücks (12).

14. Verwendung eines Rohres mit einem zumindest innenseitigen Material einer Härte von höchstens 150HV0,1 nach der Vickers-Skala als Führungsrohr (24) für eine Vorrichtung (10) nach einem der vorstehenden Ansprüche.

15. Verwendung eines Projektils (20) für eine Vorrichtung (10) nach einem der Ansprüche 1-13, insbesondere eines Metallprojektils und vorzugsweise eines unbeschichteten VA-Stahl-Projektils.
